Europäisches Patentamt

European Patent Office

Office européen des brevets

(11) Publication number: **0 224 987 B1**

# EUROPEAN PATENT SPECIFICATION

(45) Date of publication of patent specification: **15.04.92**     (51) Int. Cl.⁵: **A61K 47/36**

(21) Application number: **86306046.3**

(22) Date of filing: **05.08.86**

(54) **Drug delivery systems based on hyaluronan, derivatives thereof and their salts and method of producing same.**

(30) Priority: **29.11.85 US 804178**

(43) Date of publication of application:
**10.06.87 Bulletin 87/24**

(45) Publication of the grant of the patent:
**15.04.92 Bulletin 92/16**

(84) Designated Contracting States:
**BE CH DE FR GB IT LI NL SE**

(56) References cited:
**EP-A- 0 161 887**
**WO-A-83/00150**
**GB-A- 2 172 295**
**US-A- 4 582 865**

(73) Proprietor: **Biomatrix, Inc.**
**65 Railroad Avenue**
**Ridgefield N.J. 07657(US)**

(72) Inventor: **Balazs, Endre A.**
**200 Old Palisade Road**
**Fort Lee New Jersey 07024(US)**
Inventor: **Larsen, Nancy E.**
**1301 Summit Avenue**
**Southfield New York 10975(US)**
Inventor: **Leshchiner, Adolf**
**623 Prospect Avenue**
**Fairview New Jersey 07022(US)**

(74) Representative: **Allam, Peter Clerk et al**
**LLOYD WISE, TREGEAR & CO. Norman**
**House 105-109 Strand**
**London WC2R 0AE(GB)**

Rank Xerox (UK) Business Services

**Description**

The present invention relates to drug delivery systems based on hyaluronan or derivatives thereof such as hylan and their salts.

Hyaluronic acid ("HA") is a well known, naturally occurring polysaccharide containing alternating N-acetyl-D-glucosamine and D-glucuronic acid monosaccharide units linked with 1->4 bonds and the disaccharide units linked with 1->3 glycoside bonds. In keeping with generally accepted chemical nomenclature for polysaccharides, the term "hyaluronan" is increasingly being used in place of the traditional "hyaluronic acid". As used herein hyaluronan is intended to mean not only hyaluronic acid but also salts thereof such as the sodium salt as well as chemically modified derivatives of hyaluronan including "hylan". Strictly speaking, hylan is not hyaluronan; but for the purposes of this specification the two terms are often used interchangeably because within the context of the invention their use and application are interchangeable. Hylan is a cross-linked, but nevertheless soluble derivative of HA whose preparation is described in detail in US-A-4 713 448. Stated briefly, hylan is prepared by subjecting HA to a cross-linking reaction in situ, that is, in the animal tissue from which it is obtained before its extraction from such tissue. It is soluble notwithstanding its cross-linked nature because the degree of cross-linking is relatively low as compared with more traditional cross-linked HA. Hyaluronic acid usually occurs as the sodium salt. The molecular weight of HA is generally within the range of 50,000 to $8 \times 10^6$ and sometimes even higher.

Hyaluronic acid is one of the major components of the extracellular matrix and is found in abundance in some tissues like synovial fluid and the vitreous of eye. Hyaluronic acid and its salts give very viscous and elastic solutions in water or physiological salt solution.

Being a naturally occurring polymer, hyaluronic acid does not give a foreign body reaction when implanted into a living body and has an excellent biocompatibility. The combination of these properties together with the known viscoelasticity of HA facilitates the use of hyaluronic acid in the biomedical field. Thus, a 1% solution of sodium hyaluronate (Healon[R]) is used in eye viscosurgery (L.A. Pape and E.A. Balass, Ophthalmology, 87, No. 7, 1980). Hyaluronic acid is also used to impart biocompatibility to various polymeric materials. (E.A. Balazs and A. Leshchiner, U.S. Patent 4,500,676, 1985).

EP-A-0161887 discloses a cross-linked hyaluronic acid which is prepared by cross-linking hyaluronic acid, or salts thereof, with a polyfunctional epoxy compound. It is taught that this cross-linked product can be used for a number of different medical or cosmetic purposes, and may be employed in conjunction with substances having therapeutic activity. However, there is no suggestion in EP-A-0161887 that the cross-linked hyaluronic acid would be useful in drug delivery systems.

For completeness, there should also be mentioned the family of national patents in the designated Contracted States which are derived from US-A-4581865, and which, although not prior publications, have a priority date of 6th December 1984. These national patents teach that water-insoluble cross-linked hyaluronic gels formed using divinyl sulfone as cross-linking agent are useful in slowing the rate of drug release in drug delivery systems.

Hereinafter, the terms hyaluronic acid and hyaluronan are used interchangeably; that is, the use of either one is intended to include the other and, as previously mentioned, their use is also intended to include hylan, unless the context indicates otherwise.

The present invention is directed to new drug delivery systems based on hyaluronic acid in soluble or non-soluble cross-linked forms. In both cases, non-modified or modified hyaluronic acid serves as a vehicle which provides a slow release of a drug from a system.

In accordance with one aspect, the present invention is concerned with the use of a polymeric component as an agent for slowing the release of a substance having or pharmacological activity in the preparation of a composition for therapeutic treatment, said polymeric component being a water-soluble or water-insoluble hyaluronan or hylan, other than a water-insoluble cross-linked hyaluronan gel formed using divinyl sulfone as cross-linking agent.

Further, the present invention provides a drug delivery system comprising a polymeric component, a selected amount of at least one substance having pharmacological activity and a support or substrate for said polymeric component, wherein said polymeric component is a water-soluble or water-insoluble hyaluronan or hylan.

Methods for preparing the drug delivery products of this invention are also disclosed.

The drug delivery compositions according to the present invention include the following:

1. Hyaluronic acid solutions in which a drug substance is dissolved or dispersed;

2. A cross-linked hyaluronic acid gel forming a macromolecular "cage" in which a drug substance is dispersed;

3. A cross-linked mixed gel of hyaluronic acid and at least one other hydrophilic polymer in which a drug substance is dispersed;

4. A cross-linked gel of hyaluronic acid or cross-linked mixed gel of hyaluronic acid and at least one other hydrophilic polymer containing a drug substance which is covalently attached to the macromolecules of hyaluronic acid or the other polymer.

Any substance which has biological or pharmacological activity and which is normally considered to be a drug can be used as the drug component in the products according to the present invention. This substance can be soluble or not soluble in aqueous medium; it can be of relatively low molecular weight or it can be a polymeric drug, and the choice of the substance will clearly depend upon the specific use of the end product. It should be understood that any combination of one or more drug substances can be used in the products according to the invention.

As mentioned above, the polymeric component which imparts the drug delivering properties to the product of the invention is hyaluronan or hylan in various forms which differ in their solubility in aqueous mediums as well as combinations of hyaluronic acid with other hydrophilic polymers. We have found that solutions of hyaluronic acid can provide the above mentioned delivering properties. Hyaluronic acid, when in its most highly polymerized form, ie with a molecular weight which can exceed $8 \times 10^6$, forms solutions in water or physiological salt solutions which demonstrate outstanding viscoelasticity.

When a drug substance is dissolved or dispersed in this solution, its diffusion is substantially slower and this contributes to the delivering properties of such a system. In the case of a drug containing cationic groups, an ionic interaction can occur between hyaluronic acid macromolecules having carboxyl groups and the drug and this interaction slows down the diffusion of the drug from the system even further.

The hyaluronic acid solutions discussed hereinabove all include a so-called acid putty. (Balazs, A.E., Fed. Proc. Vol. 25, No. 6, 1817-22 (1966)). Hyaluronic acid is known to form a very elastic, almost hard body when the pH of a solution thereof is around 2.5. The hardness of this putty depends mainly upon the hyaluronic acid and sodium chloride concentrations in the solution and the temperature. This putty, despite its consistency, should be considered as a solution because it can be infinitely diluted with water to give dilute solutions with the usual properties of a solution. We have found that the ability of hyaluronic acid to form the acid putty can be conveniently used to obtain a product with drug delivering properties. It should be understood, however, that these products can only be used in certain circumstances because they are quite acidic.

The hyaluronic acid concentration in the products, based on the soluble polymers, can be in the range of from about 0.05 to 4% by weight and higher, depending on the end use of the product. The drug concentration can be varied over very broad limits and preferably should be chosen depending upon the solubility of the drug, its pharmacological activity, the desirable effect of the end product, etc. Although the above discussed products can be used as injectables, the other products according to the invention containing non-soluble hyaluronic acid are substantially more efficient as injectable drug delivery systems. For this reason, it is preferable to use soluble products for topical applications, ie as eye drops or for skin treatment.

We have found that eye drops containing hyaluronic acid remain on the surface of the eye longer and thus provide longer and more uniform action of the drug on the eye.

The other types of drug delivery products according to the invention are based on insoluble cross-linked gels of hyaluronic acid. Numerous substances can be used to cross-link hyaluronic acid including formaldehyde, epoxides, polyaziridyl compounds, divinyl sulfone (DVS) and others. We have found that the preferred cross-linking agent is divinyl sulfone; we do not claim in claim 1 the use of a water-insoluble cross-linked HA gel formed using DVS as cross-linking agent as the polymeric component for slowing drug release. Divinyl sulfone reacts readily with hyaluronic acid in aqueous alkaline solutions at room temperatures, ie about 20°C, thereby providing cross-linked HA gels. These gels swell in water and water containing media. The swelling ratio depends upon the degree of cross-linking of the gel. We have found the degree of cross-linking can be controlled by changing several factors including the molecular weight of the HA, its concentration in the reaction mixture, the alkali concentration and the polymer/DVS ratio. The reaction is very fast and in most cases a strong gel can be obtained within several minutes. The swelling ratio of these gels can be from 20 up to 8000, and more, depending upon the reaction parameters.

It has also been found that the swelling ratio of cross-linked HA gels is substantially greater than the swelling ratio of cross-linked gels of other polysaccharides obtained under the same reaction conditions. This can probably be explained by the unique nature of HA (as compared to other polysaccharides) and its water solutions. We have found that in water, a large molecule of HA forms a very flexible, long random coil which takes up an extremely large volume in the solution. For example, the specific volume of a hydrated HA molecule in a physiological salt solution is about $2\text{-}6 \times 10^3$ ml/g. That means that in a quite low concentration water solution of HA, a steric exclusion phenomenon occurs which will substantially affect not only the physico-chemical properties of the solution, but the reaction of the HA with low molecular weight substances as well. In other words, the nature of the HA solutions affects the degree of cross-linking and the behavior of the cross-linked gel in a manner quite unlike anything that occurs with other polysaccharides.

We have also found that this unique property of HA to give highly swollen cross-linked gels can be used to great advantage to effect modification of the properties of cross-linked gels made of mixtures of HA with other hydrophilic polymers. These polymers include other polysaccharides, synthetic and natural, such as hydroxyethyl cellulose, carboxymethyl cellulose, xanthan gum, glycosaminoglycans, proteins and glyco proteins of various types, such as collagen, elastin, albumin, globulin, etc, sulfated proteins, synthetic water-soluble polymers, such as polyvinyl alcohol and its co-polymers, co-polymers of poly-(hydroxyethyl) methacrylate and the like. In other words, any polymer soluble in water or aqueous alkaline solutions and containing groups capable of reacting with DVS, namely hydroxyl, amino or sulfhydryl groups, can be used to obtain highly swollen cross-linked mixed gels of HA.

Another convenient method of obtaining cross-linked hyaluronic acid or mixed hyaluronic acid and other polymer gels comprises treating dry polymer preparations, ie in the form of a film with a cross-linking agent and subsequent swelling of the product in the desired medium. Thus, a dry film made of hyaluronic acid or of a mixture of hyaluronic acid with another polymer (or polymers) can be cross-linked by treatment in solution of divinyl sulfone in a mixture of water and a solvent which cannot dissolve hyaluronic acid and which is inert towards the cross-linking agent, eg acetone. By changing the ratio between water and the organic solvent, the swelling ratio of the cross-linked film in water can be conveniently controlled. However, the swelling ratios of these cross-linked products are usually substantially less than for gels obtained by cross-linking carried out in solutions of hyaluronic acid or its mixtures with other polymers.

The cross-linked gels can be used as such or in combination with various supports or substrates, such as polymeric porous sponges, gauze, polymeric films, etc.

We have found that the above described gels of hyaluronic acid or mixed gels of hyaluronic acid with other polymers are excellent drug delivery systems when loaded with a substance or substances having pharmacological or biological activity.

The domain of the cross-linked hyaluronic acid (either alone or co-cross-linked with other polyanionic or neutral polymers) forms a so-called "molecular cage". In this cage, hydrophilic or hydrophobic molecules of various pharmacological or biological activity can be dispersed. Thus, the cage constitutes a depot for these substances of various molecular sizes. The substances contained in the domain of the molecular cage will be delivered into the environment by diffusion therefrom. The delivery process is controlled by such factors as the exclusion volume effect and the pore size of the molecular cage and by the molecular interaction between the polymeric network and the substance contained therein. Thus, the molecular cage forms a depot for the controlled delivery of drugs or other substances to the skin or other tissues.

There are several methods for combining a drug with the gel and, accordingly, several types of products which can be obtained.

One of the methods comprises diffusing a drug into a gel when the gel is put into a solution of the drug. The diffusion process is usually slow and depends upon the drug concentration, temperature of the solution, size of the gel particles, etc. The product obtained by this method is a gel in which a drug substance is uniformly dispersed.

The same type of product can be obtained by dehydrating a hyaluronic acid gel and reswelling it in a drug solution. To dehydrate a gel one can use a water-miscible organic solvent or alternatively, water from a gel can be removed by drying. However, we have found that it is preferable to use a solvent because after drying at a low or elevated temperature, the gel cannot re-swell to its initial degree of swelling. On the other hand, after dehydrating with a solvent, the gel swells to the same volume it had before the treatment. We have found that preferable solvents are ethanol and isopropanol, and ketones such as acetone, though other solvents can also be used.

Yet another method can be used to obtain products of this type. This method comprises allowing a concentrated hyaluronic acid gel resulting from a cross-linking reaction previously carried out in a relatively concentrated solution of hyaluronic acid to swell in a solution of a drug substance.

Although these three methods all result in products which are essentially the same, each of the methods has certain advantages when compared to any of the other methods for any specific product and, hence, the choice of method should be made with consideration given to such parameters as nature of the drug, the desired concentration of the drug in the system, the delivery rate, etc.

A drug delivery system of another type according to the present invention is one in which a drug is covalently attached to macromolecules of hyaluronic acid and/or other polymers forming a gel. These systems are characterized by a substantially slower rate of delivery than those described above. Delivery of

4

a drug from these systems occurs when the gel is degraded in the living body as a result of numerous metabolic processes taking place in vivo. The degradation process is usually slower than diffusion which provides the delivery of a drug in other types of products according to the present invention. Nevertheless, the rate of the degradation process can be controlled by several means, including adjusting the density of the cross-links in the gel or by co-cross-linking hyaluronic acid with polymers which can be degraded in the body more easily than hyaluronic acid, eg proteins. By changing the concentration of such polymer components in the mixed gels, one can conveniently control their rate of degradation and, thus, the rate of drug delivery.

Another possibility of drug delivery for this type of product involves the use of such chemical bonds for attachment of a drug to polymeric molecules forming a gel which have a controllable rate of hydrolysis in a physiological environment.

To obtain this type of a product one can use a drug substance which can react with a cross-linking agent. In this case, the attachment of a drug to polymers occurs in a cross-linking reaction used for the gel formation. Yet another method can be used to obtain this product. This method comprises chemically modifying a cross-linked gel after its formation, using the reactive hydroxyl groups of hyaluronic acid or the reactive groups of the polymers co-cross-linked with the hyaluronic acid to which a drug substance can be attached by numerous chemical reactions. Alternatively, additional reactive groups can be introduced by chemical treatment of a cross-linked gel which affects the macromolecules of hyaluronic acid or co-cross-linked polymers and a drug can be covalently attached to these newly formed reactive groups.

The drug delivery products according to the present invention can be used for any application where conventional drugs are used - in topical formulations for use in ophthalmology and dermatology, as injectable or implantable materials, etc. The principal advantage of these products is provided by the fact that the polymeric component of the product, namely, hyaluronic acid in soluble or insoluble form, has outstanding biocompatibility and does not cause any complications when used in humans. By being combined with other materials like polymeric substrates, sponges, gauze, etc, the drug delivery products according to the invention can be used in numerous medical devices including contraceptive devices, wound dressings, drug delivery patches, etc.

The processes by which the hereinabove described products preferably are obtained will now be described in detail.

In order to obtain a hyaluronic acid solution in which a drug substance is dissolved or dispersed any conventional method can be used. Sodium hyaluronate or hyaluronic acid from any source can be dissolved in water or in physiological saline to a desired concentration and then a drug is dissolved or dispersed in the resulting solution. Alternatively, a solution or dispersion of a drug can be mixed with hyaluronic acid solution. The polymer concentration is chosen depending upon the end use of the product and the molecular weight of hyaluronic acid. We have found that it is preferable to use a high molecular weight polymer, ie a hyaluronic acid with a molecular weight of $1 \times 10^6$ or higher. The usable concentration of hyaluronic acid of this molecular weight can vary from as low as 0.05 wt % for ophthalmic solutions to as high as 2 wt % and even higher for skin formulations. The drug concentration is chosen depending upon the desired activity of the product.

As mentioned above, the preferred cross-linking agent for obtaining cross-linked gels is divinyl sulfone. In order to obtain a cross-linked gel, a sample of sodium hyaluronate or hyaluronic acid from any source is dissolved in dilute alkaline solution. The molecular weight of HA can be from 50,000 up to $8 \times 10^6$ and even higher. The molecular weight affects the reaction - the higher the molecular weight the greater the possibility of obtaining a cross-linked gel.

The alkali concentration in the reaction mixture can be from 0.005 M to 0.5 M and higher. The lower limit is dictated by the necessity to have the pH of the medium not lower than 9 and the upper limit by the hydrolysis of HA in an alkaline solution. Usually, a decrease in alkali concentration results in gels with a greater swelling ratio, probably because a smaller amount of DVS takes part in the cross-linking reaction.

The concentration of HA in the starting solution can vary from 1% by weight up to 8% by weight and higher. When the concentration is below the lower limit, a cross-linked gel cannot be obtained even at a low HA/DVS ratio. When the concentration is too high, the solution becomes so viscous that it is difficult to handle it. The HA concentration substantially affects the swelling behavior of the gels: the swelling ratio usually decreases with HA concentration.

We have found that the HA/DVS ratio in the reaction mixture is another parameter which can be conveniently used to control the swelling ratio of the cross-linked HA gel. An increase in the ratio results in more highly swollen soft gels (the swelling ratio is about 4000 and higher), whereas hard and less swollen gels are obtained when this ratio is decreased. In general, the HA/DVS weight ratio can be from 15:1 to 1:5 and lower.

The cross-linking reaction is usually carried out at room temperature, ie about 20°C, but it can be performed at a lower or higher temperature, if desired. However, it should be kept in mind that HA degrades relatively rapidly in alkaline solutions at elevated temperatures and, if such degradation occurs, the decrease in MW can affect the properties of the obtained gels.

The cross-linking reaction is relatively fast and strong gels are formed usually in several minutes when the HA concentration is high enough and the HA/DVS ratio is low. But even at low HA concentration in the reaction mixture, the gel formation starts usually 10-15 minutes after addition of DVS. We have found that in most cases one hour is sufficient for completion of the cross-linking reaction.

To obtain mixed cross-linked gels of hyaluronic acid and other hydrophilic polymers, the same reaction conditions as for HA alone can be used. The swelling ratio of these gels can be conveniently controlled by varying the HA content in the polymer mixture. The swelling ratio is usually increased with an increase of the HA content. The composition of the polymer mixture in a mixed gel can vary over a broad range depending on the swelling ratio of the cross-linked gel, biodegradability, rate of drug delivery desired, etc. The preferred content of hyaluronic acid in the mixture is from 5 to 95 wt %. The choice of a polymer or polymers to be co-cross-linked with hyaluronic acid depends upon many factors as has already been discussed above.

To load a cross-linked swollen gel with a drug using the diffusion process, the gel can be put into a drug solution. The time for completion of this process depends upon gel particle size, gel swelling ratio, temperature of the process, stirring, concentration of the drug in the solution, etc. By proper combination of these parameters, a swollen gel can be loaded with a drug in a relatively short period of time.

We have found that good results can be obtained when a gel obtained after a cross-linking reaction is allowed to swell in a drug solution. The excess alkali which is present in the gel can be neutralized with hydrochloric acid with the formation of sodium chloride, a substance which is desirable, or at least not undesirable, in many drug delivery products.

To dehydrate a cross-linked gel with a solvent it is enough to put the gel, in any form, ie as fine particles or as a membrane, into a solvent, preferably a volatile solvent, eg isopropanol, and keep it in the solvent for a sufficient amount of time to remove water from the gel. The degree of water removal depends upon the size of the particles or the membrane thickness, the gel/solvent ratio, etc. The treatment with a solvent can be repeated several times, if desired. The solvent from the gel can be removed by drying under normal pressure or in vacuum at room or elevated temperature. The thusly dehydrated gel, when put into a drug solution, reswells to the initial swelling ratio. We have found this method to be very convenient for loading cross-linked gels with a drug.

When a drug delivery system with drug molecules covalently attached to polymers forming a gel is desired, the drug can be directly introduced into the reaction mixture during gel preparation. Essentially the same reaction conditions can be used. The suitable drug substances are those which contain chemical groups reactive towards the cross-linking agent, preferably divinyl sulfone. Examples of such chemical groups are hydroxyl, amino and sulfhydryl groups. An example of the drug which can be used in this process is the antibiotic gentamicin.

The invention is illustrated by the Examples which follows.

Example 1

This example illustrates the drug delivery capability of hyaluronic acid solutions. Fluorescein was used as a model substance to visualize the effect of hylan on tear film break-up times (B.U.T.).

An 0.1% solution of sodium hyaluronate obtained from rooster combs in 0.15 M aqueous sodium chloride was prepared.

Dry sodium fluorescein was added to this solution so that the final concentration of fluorescein was 0.125%. A solution of 0.125% sodium fluorescein in a balanced salt solution was used as a control.

Aotus trivargotus monkeys (2) were anesthetized and maintained on ketamine (12.5 mg/kg) and rompun (2 mg/kg) by intramuscular injection. Once anesthetized each monkey was immobilized. The eyelids of the eye were held open for topical instillation of one 10 $\mu$l drop of balanced salt solution (BSS) containing 0.125% sodium fluorescein. The lids were manually blinked twice to distribute the dye and then left open for a control measurement. A stopwatch was begun immediately after the second blink. The eye tear film was scanned with a broad beam slit in a darkened room using a blue filter (Nikon slit lamp) until the film began to break up and disappear. At this point the stopwatch was stopped and the time recorded.

After the control B.U.T. measurements were made, the same monkeys were used to test the 0.1% HA/0.125% fluorescein solution using the same technique as described for the control.

Values of tear film break-up time (B.U.T.) are listed in Table 1; SEM = standard error of the mean; BSS = balanced salt solution.

TABLE 1

|  | B.U.T. (sec) | SEM |
|---|---|---|
| Control BSS/0.125% fluorescein | 33 | 11 n = 9 |
| 0.1% HA opthalmic solution/ 0.125% fluorescein | 446 | 126 n = 6 |

The data indicates that the presence of HA in an eye solution increases the length of time the tear film covers the cornea, and therefore increases the availability of molecules combined with HA in this solution.

Example 2

This example illustrates the preparation of an hyaluronic acid putty containing salicylic acid.

A 2% solution of sodium hyaluronate was prepared in 0.2 N aqueous sodium hydroxide. 1.38 g (0.01 M) of salicylic acid were stirred into 50 ml of the above solution and 2.5 ml of 4 N aqueous sodium hydroxide were added to the mixture to neutralize the alkali and a slight excess of the acid (about 0.2 ml) was subsequently used to bring the pH of the mixture to about 2.5.

The product obtained consisted of a very elastic hyaluronic acid putty in which finely dispersed salicylic acid was evenly distributed. This formulation can be spread as a thin layer on the skin and used for slow delivery of salicylic acid to the skin.

Example 3

This example illustrates the slow release of radioactively labeled serotonin(N-hydroxytryptamine binoxolate, 5-[1,2 - $^3$H(N)]- from hyaluronic acid solution.

An 0.1% solution of sodium hyaluronate obtained from rooster combs in water was prepared and mixed with $^3$H-serotonin (1 $\mu$Ci/ml; final concentration of $^3$H-serotonin = 40 nM). 5 ml of the $^3$H-serotonin/0.1% sodium hyaluronate solution were placed in dialysis tubing (10,000 MW cutoff) and then placed into a beaker containing 500 ml of distilled water. A 5 ml solution of 40 nM $^3$H-serotonin in water was placed into dialysis against 500 ml distilled water in a second beaker. Triplicate 0.05 ml aliquots were removed from each beaker at regular time intervals up to 3 hours; one 24 hour aliquot was made and the contents of the dialysis bags were analysed.

For the mixture of 0.1% sodium hyaluronate and labeled drug, the rates of release ($\mu$Ci/hour/ml) and the % of release were: 0.108 (10 minutes) and 2.3%; 0.096 (60 minutes) and 14.6%; 0.084 (120 minutes) and 27%; the average rate of release over 3 hours was 0.098± 0.032 with 33.7% of the drug released; there was 20% (0.78 $\mu$Ci) of added drug remaining in the HA solution after 24 hours.

For the water solution of labeled drug the rates of release ($\mu$Ci/hr/ml) and % release were: 3.42 (10 minutes) and 14.3%; 0.204 (60 minutes) and 47.7%; 0.16 (120 minutes) and 70.4%; the average rate of release over 3 hours was 0.308± 0.192 with 82.2% of the drug released. At 24 hours 2% of the drug remained inside the dialysis bag (98% release).

The results indicate that the presence of HA in a mixture of this drug reduces the rate of release by approximately 10 fold.

Example 4

This example illustrates the preparation of hyaluronic acid immobilized in a porous polymeric sponge and delivery of a drug from this product.

0.32 g of sodium hyaluronate obtained from rooster combs (limiting viscosity number 4900 cc/g) was dissolved in 13 ml of 0.2N sodium hydroxide to give 2.5 wt % solution. A solution of 0.08 g of divinyl sulfone in 1 ml of 0.2 N sodium hydroxide was added and the mixture was stirred vigorously for about 5 minutes. Cylindrical porous sponges made of a polyurethane were dipped into the still liquid reaction mixture, squeezed in the mixture several times to remove air from the pores and left in the mixture for 5 minutes. Then the sponges were removed from the mixture and left for an hour to allow the reaction mixture to gel inside the pores. Then, the sponges with gel filling the pores were put into 0.15 M aqueous sodium chloride and kept there for 24 hours. Finally, the sponges were dried in air for 30 hours. The thusly obtained dry sponges were incubated in 5 ml of 40 nM $^3$H-serotonin (1 $\mu$Ci/ml) for 24 hours at 40°C. After this time each sponge was dipped 3 times into 10 ml of $H_2O$ and then each placed into a beaker containing 50 ml of distilled water. Triplicate 0.05 ml of aliquots of water medium were removed at regular intervals up to 96 hours and the radioactivity measured in a liquid scintillation counter. The % release of the labeled drug was determined from the total uptake.

For the sponge with immobilized hyaluronic acid gel the % release of drug at 0.5, 1, 4, 72 and 96 hours was: 3.8%, 4.9%, 8.8%, 53% and 62%, respectively. The average rate of release was 2.03%/hr/sponge ± 1.18%.

For the control (untreated sponge), the % release of drug at 0.5, 1, 4, 72 and 96 hours was: 98.7%, 45.1%, 54%, 92% and 92%, respectively. The average rate of release was 26.8%/hr/sponge ± 40.8%.

The results clearly indicate that the immobilized HA gel causes a 20 fold decrease in the average rate of release of the labeled drug and also reduces the variability in the rate of release of the drug from the sponge.

Example 5

This example illustrates the preparation of a cotton gauze with a hyaluronic acid gel film immobilized on it and delivery of a drug from this sytem.

A 0.2 wt % solution of sodium hyaluronate obtained from rooster combs (limiting viscosity number 4900 cc/g) in water-isopropanol mixture 90/10 was prepared. Pieces of bleached cotton gauze were dipped in the solution and then dried in air for 2 hours. The procedure was repeated one more time. The gauze with a polymer film on it was dipped in a mixture of the following composition, percent by weight: acetone 70, water 30, 0.2 N sodium hydroxide 4, divinyl sulfone 1.6, and kept in this mixture for 30 minutes. Then the gauze was removed from the solution, dried in air for 60 minutes and put in water to remove alkali and other soluble substances and to allow the cross-linked hyaluronic acid coating to swell. The thusly prepared gauze was air dried and cut into 1 $cm^2$ pieces. Each sample was placed in a solution of gentamicin/$^{125}$I-gentamicin; the final gentamicin concentration was 1 mg/ml. After incubation for 18-24 hours at 4°C, each 1 $cm^2$ piece was rinsed in 10 ml of 0.15 ml NaCl and placed in 20 ml of 0.15 M NaCl. 0.1 ml aliquots of medium were removed at 1, 2, 4, 24, 48 and 96 hours.

The HA/gauze sample releases 6.3% and 11.4% of the imbibed gentamicin within 24 and 96 hours. The control gauze sample released 11.2 and 20% at these same time intervals. These results indicate that by immobilizing hyaluronic acid on a cotton gauze, the rate of release of a drug such as the antibiotic gentamicin may be reduced considerably (ie about 50%).

**Claims**

1. The use of a polymeric component as an agent for slowing the release of a substance having pharmacological activity in the preparation of a composition for therapeutic treatment, said polymeric component being a water-soluble or water-insoluble hyaluronan or hylan, other than a water-insoluble cross-linked hyaluronan gel formed using divinyl sulfone as cross-linking agent.

2. The use in accordance with Claim 1, wherein the polymeric component is a water-soluble hyaluronan or hylan and comprises an aqueous hyaluronan or hylan solution.

3. The use in accordance with Claim 2, wherein the said substance is dissolved or dispersed in the aqueous solution.

4. The use in accordance with Claim 2 or Claim 3, wherein the solution is a viscoelastic putty.

5. The use in accordance with any one of Claims 2-4, wherein the hyaluronan or hylan concentration is from 0.05 to 4% by weight.

6. The use in accordance with any one of Claims 2-5, to prepare a composition in the form of an injectable product or a topical product such as pharmaceutical eye drops.

7. The use to prepare a topical product in accordance with Claim 6, wherein the hyaluronan or hylan has a molecular weight of at least $1 \times 10^6$ and the concentration of hyaluronan or hylan is from 0.05 to 2% by weight.

**8.** The use in accordance with any one of Claims 2-7, wherein the said substance is serotonin, or salicylic acid.

**9.** The use in accordance with Claim 1, wherein the hyaluronan or hylan is a water-insoluble hyaluronan or hylan, respectively.

**10.** The use in accordance with Claim 9, wherein the water-insoluble hyaluronan or hylan is a cross-linked gel of hyaluronan or hylan, respectively.

**11.** The use in accordance with Claim 9, wherein the water-insoluble hyaluronan or hylan is in the form of a molecular cage and the substance is dispersed within said molecular cage.

**12.** The use in accordance with Claim 9, wherein the said substance is covalently bonded to the macromolecules of hyaluronan or hylan.

**13.** The use in accordance with Claim 12, to prepare a gel of cross-linked hyaluronan or hylan, and gentamicin covalently attached thereto.

**14.** A drug delivery system comprising a polymeric component, a selected amount of at least one substance having biological or pharmacological activity and a support or substrate for said polymeric component, wherein said polymeric component is a water-soluble or water-insoluble hyaluronan or hylan.

**15.** A drug delivery system in accordance with Claim 14 and comprising (a) a membrane formed of a gel of hyaluronan or hylan containing gentamicin or mydriacyl, or (b) a membrane formed of a gel of hyaluronan or hylan and chondroitin sulfate containing gentamicin.

**16.** A drug delivery system in accordance with Claim 14 and comprising a porous polymeric sponge for example a polyurethane sponge, said sponge having a hyaluronan or hylan gel immobilized therein together with said substance, preferably serotonin.

**17.** A drug delivery system in accordance with Claim 14 and comprising a cotton gauze, said gauze having a hyaluronan or hylan gel immobilized therein together with said substance, preferably gentamicin.

**18.** A method of obtaining a product as defined in Claim 2, comprising either (a) dissolving or dispersing the said substance in a water or saline solution of hyaluronan or hylan, or (b) mixing a solution or dispersion of the substance with a hyaluronan or hylan solution.

**19.** A method of obtaining a product as defined in Claim 4, comprising adding the said substance to a solution of hyaluronan or hylan and adjusting the pH of the resulting mixture to about 2.5.

**20.** A method of obtaining a product as defined in Claim 10, comprising (a) placing the gel into a solution of the said substance and allowing the substance to diffuse into the gel whereby a product having the substance uniformly dispersed therethrough is obtained; (b) dehydrating the gel and placing the dehydrated gel into a solution of the substance to cause reswelling of the dehydrated gel, said substance being diffused into the gel while the reswelling occurs; or (c) placing a concentrated gel in a solution of said substance and allowing the gel to swell in said solution whereby the substance is diffused into the gel while it is swelling.

**21.** A method in accordance with Claim 20 wherein said dehydrating is effected by treating the gel with a water miscible solvent, preferably ethanol, isopropanol or acetone, or by drying.

**Revendications**

**1.** Exploitation d'un élément polymère comme agent retardateur de libération d'une substance comportant une activité pharmacologique dans la préparation d'une composition de traitement thérapeutique, ledit élément polymère étant un hyaluronane ou hylane soluble ou insoluble à l'eau, autre qu'un gel d'hyaluronane réticulé insoluble à l'eau formé à partir de sulfone divinylique comme agent réticulant.

**2.** Exploitation telle qu'à la revendication 1, selon laquelle le composé polymère est un hyaluronane ou hylane soluble à l'eau et comporte une solution aqueuse d'hyaluronane ou d'hylane.

**3.** Exploitation telle qu'à la revendication 2, selon laquelle ladite substance est dissoute ou dispersée dans la solution aqueuse.

**4.** Exploitation telle qu'à la revendication 2 ou la revendication 3, la solution étant sous forme de mastic viscoélastique.

**5.** Exploitation selon l'une ou l'autre des revendications 2-4, dont la concentration d'hyaluronane ou d'hylane est de l'ordre de 0,05 à 4% par poids.

**6.** Exploitation selon l'une ou l'autre des revendications 2-5, pour la préparation d'une composition sous forme de produit injectable ou produit topique tel que les gouttes pharmaceutiques pour les yeux.

**7.** Exploitation selon la revendication 6, selon laquelle le poids moléculaire de l'hyaluronane ou de l'hylane est de $1 \times 10^6$ au minimum, et la concentration d'hyaluronane ou d'hylane est de 0,05 à 2% par poids.

**8.** Exploitation selon l'une ou l'autre des revendications 2-7, dont ladite substance est la sérotonine ou l'acide salicylique.

**9.** Exploitation selon la revendication 1, dont l'hyaluronane ou l'hylane est respectivement un hyaluronane ou hylane insoluble à l'eau.

**10.** Exploitation selon la revendication 9, dont l'hyaluronane ou l'hylane insoluble à l'eau est respectivement un gel réticulé d'hyaluronane ou l'hylane.

**11.** Exploitation selon la revendication 9, dont l'hyaluronane ou l'hylane insoluble à l'eau est sous forme de cage moléculaire dans laquelle la substance est dispersée.

**12.** Exploitation selon la revendication 9, dont ladite substance est en liaison covalente avec les macromolécules d'hyaluronane ou l'hylane.

**13.** Exploitation selon la revendication 12, pour la préparation d'un gel réticulé d'hyaluronane ou d'hylane et en liaison covalente avec la gentamicine.

**14.** Système d'apport de médicament comportant un élément polymère, un montant sélectionné d'une substance au minimum ayant une activité biologique ou pharmacologique et un support ou substrat pour ledit élément polymère, dont l'élément polymère est l'hyaluronane ou l'hylane soluble ou insoluble à l'eau.

**15.** Système d'apport de médicament selon la revendication 14 et comportant (a) une membrane formée à partir d'un gel d'hyaluronane ou d'hylane ayant une teneur de gentamycine ou de mydriacyle, ou (b) une membrane formée à partir d'un gel d'hyaluronane ou d'hylane et de sulphate de chondroitine à teneur de gentamicine.

**16.** Système d'apport de médicament selon la revendication 14 et comportant une éponge poreuse polymère telle qu'une éponge de polyuréthane, ladite éponge retenant un gel fixe d'hyaluronane ou d'hylane avec ladite substance, de préférence la sérotonine.

**17.** Système d'apport de médicament selon la revendication 14 et comportant une gaze de coton, ladite gaze retenant un gel fixe d'hyaluronane ou d'hylane avec ladite substance, de préférence la gentamicine.

**18.** Méthode pour obtenir un produit tel que défini à la revendication 2, comportant soit (a) la dissolution ou la dispersion de ladite substance dans une solution aqueuse ou saline d'hyaluronane ou d'hylane, soit (b) le mélange d'une solution ou dispersion de la substance avec une solution d'hyaluronane ou d'hylane

**19.** Méthode pour obtenir un produit tel que défini à la revendication 4, comportant l'apport de ladite substance à une solution d'hyaluronane ou d'hylane et l'ajustage à env. 2,5 du pH du mélange qui en résulte.

**20.** Méthode pour obtenir un produit tel que défini à la revendication 10, comportant (a) la mise du gel dans une solution de ladite substance permettant sa diffusion dans le gel, obtenant ainsi un produit dispersé à l'intérieur de manière uniforme; (b) la déshydratation du gel et la mise du gel déshydraté dans une solution de la substance provoquant le gonflement à nouveau du gel déshydraté, ladite substance étant diffusée dans le gel lors du nouveau gonflement; ou (c) la mise d'un gel concentré dans une solution de ladite substance permettant le gonflement du gel dans la solution comportant la diffusion de la substance dans le gel lors du gonflement.

**21.** Méthode selon la revendication 20, selon laquelle ladite déshydratation est effectuée par le traitement du gel avec un solvant soluble à l'eau, de préférence l'alcool éthylique, isopropylique ou l'acétone, ou par le séchage.

**Patentansprüche**

**1.** Verwendung einer polymeren Komponente als Mittel zum Verlangsamen der Freigabe eines Stoffes, der pharmakologische Aktivität bei der Herstellung einer Zusammensetzung für therapeutische Behandlung hat, wobei die polymere Komponente ein wasserlösliches oder wasserunlösliches Hyaluronan oder Hylan ist, das von einem wasserunlöslichen vernetzten Hyaluronangel, das unter Verwendung von Divinylsulfon als vernetzendes Mittel gebildet wird, verschieden ist.

**2.** Verwendung gemäss Anspruch 1, in der die polymere Komponente ein wasserlösliches Hyaluronan oder Hylan ist, und eine wässrige Hyaluronan- oder Hylanlösung enthält.

**3.** Verwendung gemäss Anspruch 2, in der der Stoff in der wässrigen Lösung aufgelöst oder dispergiert wird.

**4.** Verwendung gemäss Anspruch 2 oder Anspruch 3, in der die Lösung ein viskoelastischer Kitt ist.

**5.** Verwendung gemäss einem der Ansprüche 2 - 4, in der die Hyaluronan- oder Hylankonzentration zwischen 0,05 und 4 Gewichts% liegt.

**6.** Verwendung gemäss einem der Ansprüche 2 - 5, um eine Zusammensetzung in der Gestalt eines injizierbaren Produktes oder eines örtlich anwendbaren Produktes wie pharmazeutische Augentropfen herzustellen.

**7.** Verwendung zur Herstellung eines örtlich anwendbaren Produktes gemäss Anspruch 6, in der das Hyaluronan oder Hylan ein Molekulargewicht von wenigstens $1 \times 10^6$ hat, und die Konzentration von Hyaluronan oder Hylan zwischen 0,05 und 2 Gewichts% liegt.

**8.** Verwendung gemäss einem der Ansprüche 2 - 7, in der der Stoff Serotonin oder Salizylsäure ist.

**9.** Verwendung gemäss Anspruch 1, in der das Hyaluronan oder Hylan jeweils ein wasserunlösliches Hyaluronan oder Hylan ist.

**10.** Verwendung gemäss Anspruch 9, in der das wasserunlösliche Hyaluronan oder Hylan ein vernetztes Gel von jeweils Hyaluronan oder Hylan ist.

**11.** Verwendung gemäss Anspruch 9, in der das wasserunlösliche Hyaluronan oder Hylan in der Gestalt eines Molekularkäfigs ist, und der Stoff in dem Molekularkäfigs dispergiert ist.

**12.** Verwendung gemäss Anspruch 9, in der der Stoff kovalent an die Makromoleküle von Hyaluronan und Hylan gebunden ist.

**13.** Verwendung gemäss Anspruch 12 zum Herstellen eines Gels aus vernetztem Hyaluronan oder Hylan, und kovalent daran gebundenem Gentamicin.

14. Arzneistoffabgabesystem, das eine polymere Komponente einschliesst, einen ausgewählten Betrag wenigstens eines Stoffes, der biologische oder pharmakologische Aktivität hat, und einen Träger oder Substrat für die polymere Komponente, worin die polymere Komponente ein wasserlösliches oder wasserunlösliches Hyaluronan oder Hylan ist.

15. Arzneistoffabgabesystem gemäss Anspruch 14, und das (a) eine aus einem Gel von Hyaluronan oder Hylan gebildete Membran, die Gentamicin oder Mydriacyl enthält, oder (b) eine aus einem Gel von Hyaluronan oder Hylan und Gentamicin enthaltendes Chondroitinsulfat gebildete Membran, einschliesst.

16. Arzneistoffabgabesystem gemäss Anspruch 14, und das einen porösen polymeren Schwamm einschliesst, zum Beispiel einen Polyurethanschwamm, wobei der Schwamm ein darin immobilisiertes Gel zusammen mit dem Stoff, vorzugsweise Serotonin hat.

17. Arzneistoffabgabesystem gemäss Anspruch 14, und das eine Baumwollgaze einschliesst, wobei die Gaze ein darin zusammen mit dem Stoff, vorzugsweise Gentamicin, immobilisiertes Hyaluronan- oder Hylangel hat.

18. Verfahren zum Erhalten eines Produktes nach Anspruch 2, das entweder (a) Auflösen oder Dispergieren des Stoffes in einer Wasser- oder Salzlösung von Hyaluronan oder Hylan, oder (b) Mischen einer Lösung oder Dispersion des Stoffes mit einer Hyaluronan- oder Hylanlösung einschliesst.

19. Verfahren zum Erhalten eines Produktes nach Anspruch 4, das einschliesst, den Stoff einer Lösung von Hyaluronan oder Hylan zuzusetzen, und den pH-Wert der sich ergebenden Mischung auf ungefähr 2,5 einzustellen.

20. Verfahren zum Erhalten eines Produktes nach Anspruch 10, das folgendes einschliesst: (a) das Gel in eine Lösung des Stoffes zu bringen und zuzulassen, dass der Stoff in das Gel diffundiert, wobei ein Produkt erhalten wird, worin der Stoff gleichmässig dispergiert ist; (b) das Gel zu dehydrieren und das dehydrierte Gel in eine Lösung des Stoffes zu bringen, um zu verursachen, dass das dehydrierte Gel wiederaufgeschwellt wird, der Stoff in das Gel diffundiert wird, während die Wiederaufschwellung stattfindet; oder (c) ein konzentriertes Gel in eine Lösung des Stoffes zu bringen und zuzulassen, dass das Gel in der Lösung aufschwillt, wobei der Stoff in das Gel diffundiert wird, während es aufschwillt.

21. Verfahren gemäss Anspruch 20, in dem die Dehydrierung durch Behandlung des Gels mit einem in Wasser mischbaren Lösungsmittel, vorzugsweise Ethanol, Isopropanol oder Aceton, oder durch Trocknen bewirkt wird.